# EUROPEAN PATENT APPLICATION

(11) **EP 4 470 584 A1**
(43) Date of publication of application: **04.12.2024**
(21) Application number: 23176426.7
(22) Date of filing: 31.05.2023
(51) Int. Cl.: A61M 5/32, A61M 5/31

(54) **MEDICAL INJECTION DEVICE COVER WITH OPTIMALLY ORIENTED RFID TAG**

(71) Applicant: Becton, Dickinson and Company, Franklin Lakes, NJ 07417-1880 (US)
(72) Inventor: DELEUIL, Nicolas, 38100 Grenoble (FR); RIVIER, Cédric, 38340 VOREPPE (FR); THUILLIEZ, Julien, 38240 MEYLAN (FR); LEIBBRAND, Alfred, 38640 Claix (FR); EUVRARD, Nicolas, London SW17 0JF (GB)
(74) Representative: Germain Maureau

(57) **Abstract**

Provided herein is a cover for a medical injection device, with the cover including an outer casing and an inner casing, with the inner casing defining a cavity configured to receive at least part of a hub portion of the medical injection device and form a seal therewith. An RFID tag is integrated within the cover and includes an RFID chip and an RFID antenna operatively connected to the RFID chip. The RFID antenna includes a first tag dipole provided on a first side of the RFID chip and a second tag dipole provided on a second side of the RFID chip. The RFID tag is oriented such that a dipole axis of the RFID tag is approximately aligned with a longitudinal axis of the cover.

## Description

### BACKGROUND OF THE INVENTION

### Field of the Invention

The present disclosure relates generally to closures covers for medical injection devices and, more particularly, to medical injection device covers with an integrated RFID tag.

### Description of Related Art

Medical injection devices, such as syringes, are used in a variety of environments for administering liquids (e.g., medications or drugs) to a patient. Many syringes are provided as prefilled syringes, which provide the convenience of rapidly delivering the liquid therein to a patient without the need to first draw the medication from another container and meter its volume. For dispensing fluids, the prefilled syringe will typically include a syringe barrel with a plunger assembly inserted through an open proximal end of the barrel and an opening at the opposite distal end adapted to receive a needle therein by which a fluid is injected into the patient.

There is an increasing need for individual traceability of syringes and other medical injection devices from the manufacturing process until the final labeling, the final use, or the disposal of the medical containers. Some prior attempts to address this need for traceability along the supply chain have implemented printed machine-readable unique identifier codes on an external surface of the syringe or a remotely readable electronic component such as an RFID tag (including a RFID chip and a RFID antenna) for remote identification of the medical container. However, existing solutions for providing traceability suffer from deficiencies that may impact the ability to provide reliable tracking. As one example, machine-readable unique identifier codes that are printed on an external surface of the syringe are susceptible to wear and damage to the codes that result from handling or use of the receptacle, and may not be visible to a user when placed within sealed packaging. As another example, existing syringe designs that incorporate an RFID tag therein suffer from a complex/costly manufacturing process and/or limitations on the reading of the RFID tag by an associated reader due to limitations on the tag design or on the manner in which the tag is incorporated into the syringe.

Accordingly, a need exists in the art for a device that that addresses the above-mentioned drawbacks by providing an effective and dependable means for traceability of a syringe or other medical injection device without limitations on visual inspection or data transmission level, and that is not susceptible to damage or that causes challenges in the manufacturing process.

### SUMMARY OF THE INVENTION

Provided herein is a cover or cap for use with a medical injection device having a hub portion at a distal end thereof. The cover includes an outer casing with an inner wall having a circumference, and an inner casing at least partly inserted within the outer casing and at least partially in contact with the inner wall of the outer casing, with the inner casing including an outer wall having a circumference and the inner casing defining a cavity configured to receive at least part of the hub portion and form a seal therewith. The cover also includes an RFID tag integrated within the cover, with the RFID tag including an RFID chip and an RFID antenna operatively connected to the RFID chip, with the RFID antenna including a first tag dipole provided on a first side of the RFID chip and a second tag dipole provided on a second side of the RFID chip. The RFID tag is oriented such that a dipole axis of the RFID tag is approximately aligned with a longitudinal axis of the cover.

In certain configurations, the dipole axis of the RFID tag is an axis extending between a midpoint of the first tag dipole and a midpoint of the second tag dipole.

In certain configurations, wherein the dipole axis is aligned within +/- 20 degrees with the longitudinal axis of the cover.

In certain configurations, the dipole axis is aligned at 0 degrees with the longitudinal axis of the cover.

In certain configurations, the RFID tag is a flat RFID inlay integrated within the cover to match the curvature thereof, with the RFID inlay including a first edge and an opposing second edge, with the RFID inlay inlaid within the cover such that a gap is present between the first and second edges.

In certain configurations, the RFID tag is located within the inner wall of the outer casing, within the outer wall of the inner casing, or between the outer casing and the inner casing.

In certain configurations, the RFID tag is an Ultra High Frequency (UHF) RFID tag.

In certain configurations, the outer cover is formed of a rigid material and wherein the inner cover is formed of a deformable material.

Also provided herein is a medical injection device that includes a hub portion and a cover. The cover includes an outer casing with an inner wall having a circumference, and an inner casing at least partly inserted within the outer casing and at least partially in contact with the inner wall of the outer casing, with the inner casing including an outer wall having a circumference and the inner casing defining a cavity configured to receive at least part of the hub portion and form a seal therewith. The cover also includes a RFID tag integrated within the cover, with the RFID tag including an RFID chip and an RFID antenna operatively connected to the RFID chip, with the RFID antenna including a first tag dipole provided on a first side of the RFID chip and a second tag dipole provided on a second side of the RFID chip. The RFID tag is oriented such that a dipole axis of the RFID tag is approximately aligned with a longitudinal axis of the cover.

Also provided herein is a method of manufacturing and reading a medical injection device. The method includes providing a medical injection device including a hub at a distal end thereof and coupling a cover to the hub, the cover including an outer casing with an inner wall having a circumference, an inner casing at least partly inserted within the outer casing and at least partially in contact with the inner wall of the outer casing, with the inner casing including an outer wall having a circumference and the inner casing defining a cavity configured to receive at least part of the hub portion and form a seal therewith, and a RFID tag integrated within the cover, the RFID tag including an RFID chip and an RFID antenna operatively connected to the RFID chip, the RFID antenna including a first tag dipole provided on a first side of the RFID chip and a second tag dipole provided on a second side of the RFID chip, wherein the RFID tag is oriented such that the a dipole axis of the RFID tag is approximately aligned with a longitudinal axis of the cover. The method also includes reading the RFID tag via an RFID reader, as the medical injection device and the cover coupled thereto are conveyed along a conveyor line, so as perform a quality control check of the RFID tag.

In certain configurations, the method includes positioning the RFID reader relative to the RFID tag such that a first reader dipole and a second reader dipole of a near-field electrical antenna in the RFID reader are aligned with the first tag dipole and the second tag dipole.

In certain configurations, reading of the RFID tag includes emitting a near-field electrical field from the RFID reader to read the RFID tag.

In certain configurations, in conveying the medical injection device and the cover coupled thereto, a spacing of the medical injection device with other adjacent medical injection devices being conveyed is 30 mm or less, and wherein reading of the RFID tag via the near-field electrical field emitted from the RFID reader prevents cross-reading with one of the other adjacent medical injection devices.

In certain configurations, the dipole axis is aligned within +/- 20 degrees with the longitudinal axis of the cover, and is preferably aligned at 0 degrees with the longitudinal axis of the cover.

### BRIEF DESCRIPTION OF THE DRAWINGS

FIG. 1 is a perspective view of a syringe, with which embodiments of the disclosure may be implemented;
FIG. 2 is an exploded view of the syringe of FIG. 1;
FIG. 3 is a side view of a cover included in the syringe of FIG. 1, shown coupled to a hub portion of the syringe of FIG. 1, according to a non-limiting embodiment described herein;
FIG. 4 is a side cross-sectional view of the cover of FIG. 3;
FIG. 5 is a side view of the cover of FIG. 3 with the RFID tag thereof exploded out, to illustrate orientation thereof relative to the cover, according to a non-limiting embodiment described herein;
FIG. 6 is a side view of the cover of FIG. 3 with the RFID tag thereof exploded out, to illustrate orientation thereof relative to the cover, according to another non-limiting embodiment described herein;
FIG. 7 is a is a side view of the cover of FIG. 3 with the RFID tag thereof exploded out, to illustrate orientation thereof relative to the cover, according to another non-limiting embodiment described herein;
FIG. 8 is an exploded perspective view of a syringe, with which embodiments of the disclosure may be implemented; and
FIG. 9 illustrates a manufacturing environment via which a method of manufacturing and reading of the syringe of FIG. 1 may be performed, according to a non-limiting embodiment described herein.

### DESCRIPTION OF THE INVENTION

The following description is provided to enable those skilled in the art to make and use the described embodiments for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

In the present disclosure, the distal end of a component or of a device means the end furthest away from the hand of the user and the proximal end means the end closest to the hand of the user, when the component or device is in the use position, i.e., when the user is holding a syringe in preparation for or during use. Similarly, in this application, the terms "in the distal direction" and "distally" mean in the direction toward the distal tip of the syringe, and the terms "in the proximal direction" and "proximally" mean in the direction opposite the direction of the distal tip of the syringe.

Aspects and embodiments of the disclosure are directed to a cover for a medical injection device, with the cover including an RFID tag integrated therewith that is oriented to improve tag readability and performance.

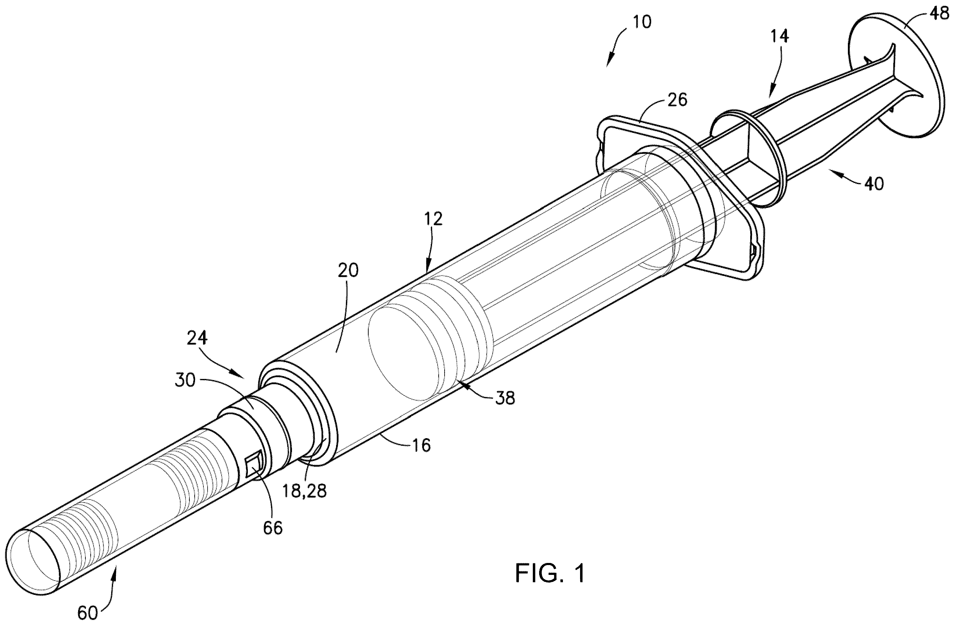

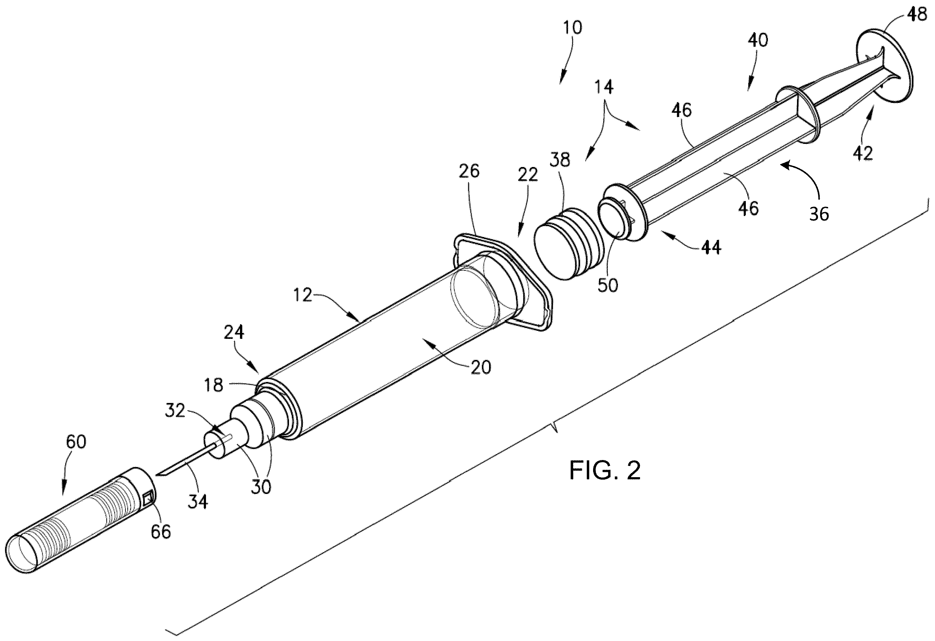

Referring to FIGS. 1 and 2, shown is a non-limiting embodiment of a medical injection device with which aspects or embodiments of the disclosure may be implemented. While the medical injection device is shown and described hereafter as a syringe ("syringe 10"), it is recognized that other medical injection devices may incorporate aspects of the disclosure, as described in detail here below.

As shown in FIGS. 1 and 2, the syringe 10 generally includes a syringe barrel 12 and a plunger assembly 14. The plunger assembly 14 is movable within the syringe barrel 12 along a longitudinal axis to an advanced position to facilitate administering of an injectable fluid (e.g., medication) to a patient, for example. The syringe barrel 12 is formed of a generally cylindrical outer wall 16 and an end member 18 that collectively define a chamber 20 for retaining fluid therein. The syringe barrel 12 includes an open proximal end 22 configured to receive the plunger assembly 14 therein and a distal end 24 at which end member 18 is positioned. The proximal end 22 of the syringe barrel 12 may include a flange 26 to facilitate handling and positioning of the syringe 10 and to maintain the relative position of the syringe barrel 12 with respect to the plunger assembly 14 during medication administration. At the distal end 24, the end member 18 may include a shoulder 28 which narrows with respect to the cylindrical outer wall 16, as well as a hub portion 30 extending out distally from shoulder 28. The hub portion 30 is formed as a partially hollow member that defines a channel 32 therethrough in fluid communication with the chamber 20. A needle 34 is attached to the hub portion 30 within channel 32, such as by being glued or otherwise secured to the hub portion 30.

The plunger assembly 14 of syringe 10 is formed of an elongate plunger rod 36 (more generally "plunger 36," as used hereafter) and a plunger head or stopper 38. The plunger 36 may include a main body 40 extending between a plunger proximal end 42 and a plunger distal end 44. In some embodiments, the main body 40 may include a plurality of elongate vanes or walls 46 extending axially along a length thereof between the plunger proximal end 42 and the plunger distal end 44. A thumb press 48 is positioned at the plunger proximal end 42 that may be engaged by a thumb (or other finger) of the user to apply a distally directed force to the plunger assembly 14 to move the plunger 36 with respect to the syringe barrel 12. In some embodiments, a flanged extension member 50 (e.g., disc-shaped flange) is positioned at the plunger distal end 44 that is configured to mate with the stopper 38. In other embodiments, the plunger distal end 44 may include a female receptacle formed therein that is configured to receive and connect to a protrusion (e.g., pin) extending out proximally from the stopper, with the protrusion and receptacle engaging via threading, for example.

The stopper 38 of plunger assembly 14 is positioned at the plunger distal end 44 so as to be movable along with the plunger 36 within the chamber 20 of syringe barrel 12. The stopper 38 may be made from a material that is different from the material of the plunger 36 and that is capable of forming a tight seal with the syringe barrel 12 as it is advanced therethrough. In some embodiments, the stopper 38 includes a receptacle (not shown) formed therein that is sized and configured to receive the flanged extension member 50 of plunger 36, with the flanged extension member 50 coupling with the receptacle via a press fit connection, for example, to secure the stopper 38 to the plunger 36, although it can be appreciated that the stopper 38 and plunger distal end 44 can be secured by other techniques known in the art.

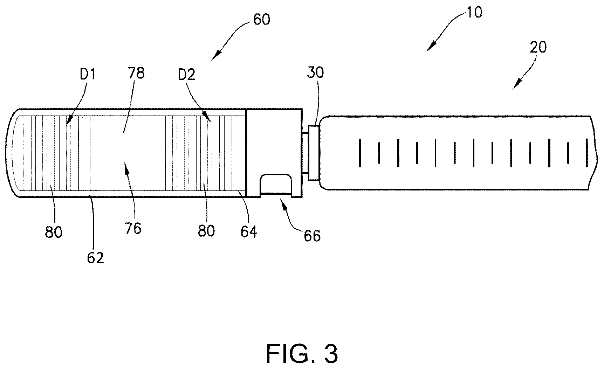

As shown in FIGS. 1 and 2, and now also in FIGS. 3-7, syringe 10 further includes a cover or cap 60 that may be coupled to the hub portion 30 of syringe barrel 12 to protect the needle 34. According to aspects and embodiments of the disclosure, and as will be explained in further detail below, the cover 60 includes an RFID tag integrated therein that allows for identification and/or tracking of the syringe 10. That is, the RFID tag may include or store information thereon regarding the contents of the syringe 10 (i.e., the medication or drug included therein, if a prefilled syringe) and the manufacturing history of the syringe and/or may provide location information to an associated reader, so as to enable tracking of the syringe 10.

The cover 60 is shown in further detail in FIGS. 3-7 according to aspects or embodiments of the disclosure. The cover 60 comprises an outer casing 62 and an inner casing 64. The outer casing 62 is typically made of a rigid material while the inner casing 64 is made of a soft material, such that the needle 34 may be safely housed within (or engage with) the cover 60 when the cover 60 is mounted on the hub portion 30 of the syringe 10. In various embodiments, the outer casing 62 may be made of polypropylene (PP), polyethylene (PE), polyethylene terephthalate (PET), polystyrene (PS) or polycarbonate (PC), preferably of polypropylene, while the inner casing 64 is made of a deformable material such as a thermoplastic elastomer (TPE) or rubber.

According to an embodiment, the outer casing 62 includes an at least partially closed distal end and an opened proximal end, and the inner casing 64 includes a closed distal end and an opened proximal end. The outer casing 62 may be manufactured by over-molding or injection molding, and may be manufactured so that, at its proximal end, the outer casing 62 optionally includes a locking window 66 that defines a through-aperture configured to receive a radial lug 68 of the inner casing 64. When present, the radial lug 68 of inner casing 64 engages with the window 66 of the outer casing 62. The outer casing 62 can also include abutment portions 70 which are positioned next to locking window 66, allowing to lock the inner casing 64 as the radial lug 68 engages into the locking window 66.

The outer casing 62 includes an inner wall 72, which may receive an outer wall 74 of the inner casing 64. Both the inner wall 72 of the outer casing 62 and the outer wall 74 of the inner casing 64 may have a cylindrical cross-section. The inner casing 64 also includes an inner wall (not shown) which is intended to cooperate with the hub portion 30 of the syringe 10 when the cover 60 is mounted on the hub portion 30. The inner wall of the inner casing 64 is intended to form a seal with an outer surface of the hub portion 30 of the syringe 10 when engaged therewith. That is, the inner casing 64 may define a cavity 75 within which receive at least part of the hub portion 30 is received and a seal formed therewith, with the cavity also housing the needle 34.

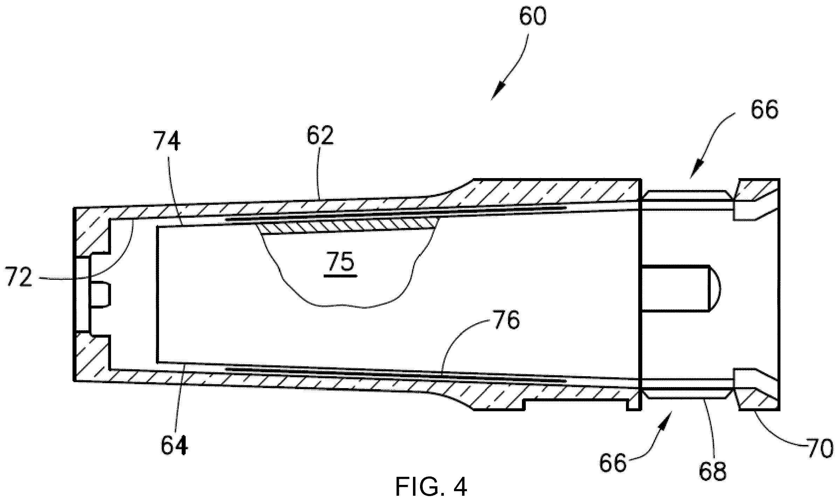

In accordance with aspects of the disclosure, and as shown in FIGS. 3 and 4, an RFID tag 76 is integrated into the cover 60. In accordance with some embodiments, the RFID tag 76 is disposed between the outer wall 74 of the inner casing 64 and the inner wall 72 of the outer casing 62. The RFID tag 76 may be in a form of a dry inlay or a wet inlay. When the RFID tag 76 is in a form a wet inlay, the RFID tag 76 may be bonded to the inner casing 64 (i.e., to the outer wall 74 thereof) or to the outer casing 62 (i.e., to the inner wall 72 thereof) by adhesive bonding. Otherwise, the RFID tag 76 may be fixed to the inner casing 64 or outer casing 62 by in-mold labeling, by adhesive bonding, by assembly, by co-molding or by over-molding. In accordance with other embodiments, the RFID tag 76 may be fixed within the inner wall 72 of the outer casing 62 or within the outer wall 74 of the inner casing 64. That is, the RFID tag 76 may be fixed within the inner wall 72 of the outer casing 62 by in-mold labelling or within the outer wall 74 of the inner casing 64 by in-mold labelling or adhesive bonding. For example, when the second material of the inner casing 64 is a TPE, the RFID tag 76 is fixed within the outer wall 74 of the inner casing 64 by in-mold labelling, and when the second material of the inner casing 64 is a rubber, the RFID tag 76 is fixed within the outer wall 74 of the inner casing 64 by adhesive bonding.

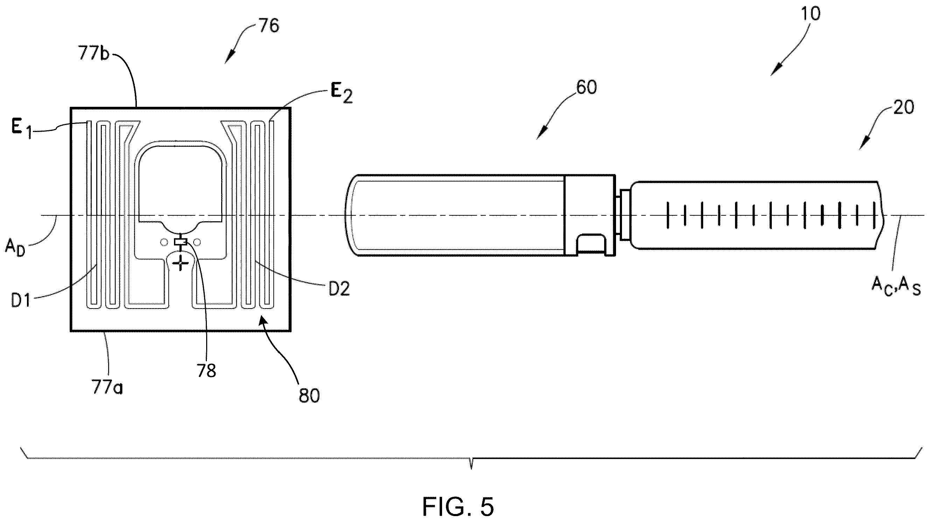

In accordance with some embodiments, the RFID tag 76 may be shaped/configured as a flat square/rectangular inlay having a first edge 77a and an opposing second edge 77b (see FIG. 5, for example). The RFID tag 76 is integrated into the cover 60 (e.g., between outer wall 74 of the inner casing 64 and the inner wall 72 of the outer casing 62) to match a curvature of the surface(s) or wall(s) to or within which it is disposed, and such that a gap is present between the first and second edges 77a, 77b. That is, the RFID tag 76 extends on less than 100% of the whole circumference of the wall 72, 74 to or within which it is disposed, such that the two opposite edges 77a, 77b of the RFID tag 76 are not in contact.

According to embodiments, the RFID tag 76 includes an RFID chip 78 connected to an RFID antenna 80. In some embodiments, the RFID tag 76 is an Ultra-High Frequency (UHF) RFID tag with frequencies of about 800-1000MHz. The UHF RFID tag 76 is readable by an associated RFID reader at a distance of up to about fifteen meters, for example. As shown in FIG. 3 and in FIGS. 5-7, RFID antenna 80 of RFID tag 76 may include two legs or dipoles D1, D2. Dipole D1 has an extremity El, and dipole D2 has an extremity E2. The RFID tag 76 is disposed between these two extremities El, E2 of the dipoles D1, D2, such as being centered therebetween or in another appropriate location between the extremities El, E2 of the dipoles D1, D2. Each of the dipoles D1, D2 of the RFID antenna 80 may be made of a plurality of square-shaped sinusoids having the same amplitude.

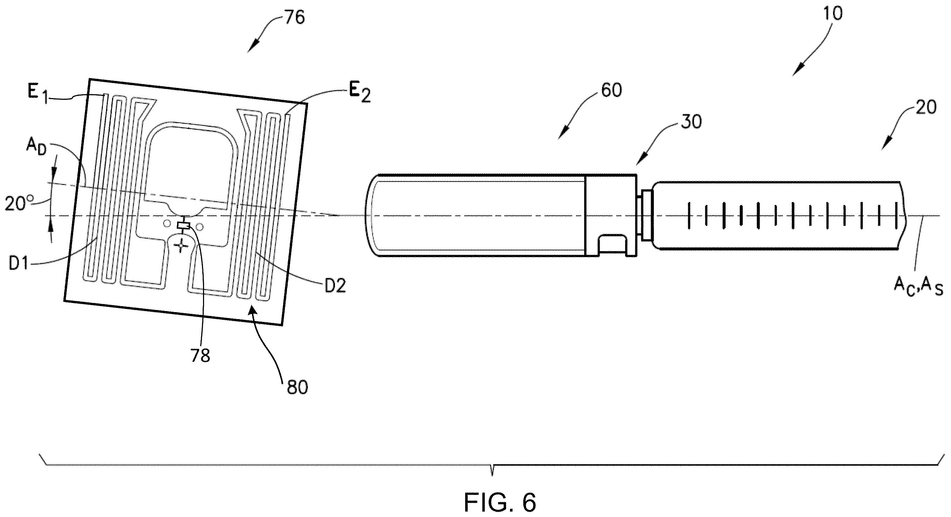

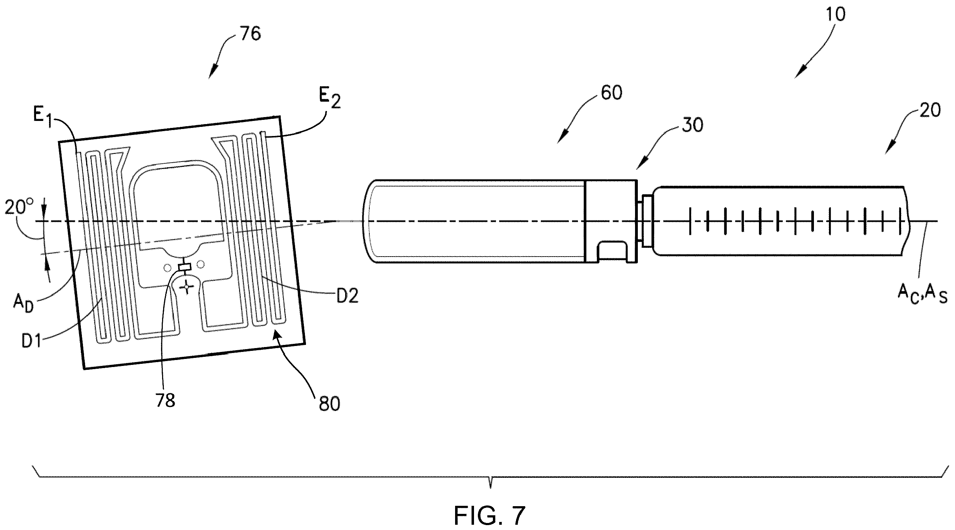

According to aspects of the disclosure, and as best shown in FIGS. 5-7 where the RFID tag 76 is shown exploded out from the remainder of cover 60, the RFID tag 76 is positioned and oriented within cover 60 such that a dipole axis, A_{D}, of the RFID tag 76 is approximately aligned with a longitudinal axis, Ac, of the cover 60 (and a longitudinal axis, As, of the syringe 10). The dipole axis A_{D} of the RFID tag 76 is defined as an axis extending between midpoints 82 of the first tag dipole D1 and the second tag dipole D2. According to embodiments, the dipole axis A_{D} is aligned within +/- 45 degrees of the longitudinal axis Ac of the cover 60. More preferably, the dipole axis A_{D} of the RFID tag 76 is aligned within +/- 30 degrees of the longitudinal axis Ac of the cover 60, and even more preferably, the dipole axis A_{D} of the RFID tag 76 is aligned within +/- 20 degrees of the longitudinal axis Ac of the cover 60 (FIGS. 6 and 7) and, still more preferably, is aligned at 0 degrees (i.e., in parallel) to the longitudinal axis Ac of the cover 60 (FIG. 5). With the dipole axis A_{D} of the RFID tag 76 aligned with the longitudinal axis Ac of the cover 60 (and syringe axis As) in this manner, performance of the RFID tag 76 may be optimized - with reading efficiency of the RFID tag 76 maximized such that the read range of the RFID tag 76 is extended and reading is enabled over a full RFID UHF bandwidth (800 to 1000MHz), and with tag orientation sensitivity being minimized such that the RFID tag 76 provides for omni-directional tag readability all around the syringe axis A_{S} (for both ETSI and FCC frequencies). Additionally, with the dipole axis A_{D} of the RFID tag 76 aligned with the longitudinal axis Ac of the cover 60 (and syringe axis As), more strict quality controls may be implemented during manufacturing/assembly of the syringe 10 and the cap 60 thereof, as explained in further detail below.

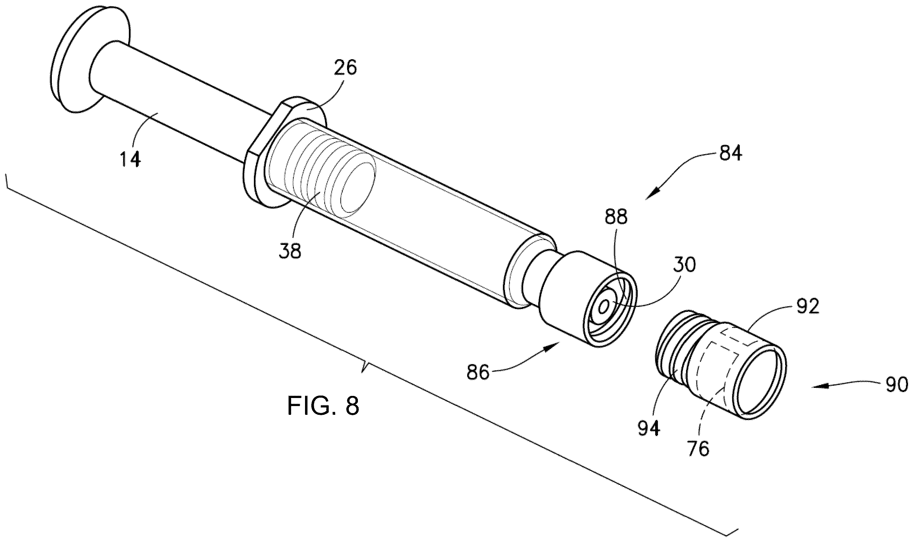

Referring now to FIG. 8, a syringe 84 is illustrated in accordance with another aspect of the disclosure. The syringe 84 is constructed similar to the syringe 10, except that the syringe 84 includes a luer fitting at the distal end 24 thereof rather than a needle. That is, the distal end 24 of the syringe 84 includes an adaptor 86 that may be mounted around the hub portion 30 in such a way that an annular space is created between the adaptor 86 and the hub portion 30. The adaptor 86 includes an internal thread 88 thereon by which a cover 90 may be connected to the adaptor 86 and over hub portion 30. In some embodiments, adaptor 86 may provide a luer connection to the syringe 84.

According to aspects of the disclosure, cover 90 may have a structure similar to the cover 60 previously described - with the cover 90 including an outer casing 92 and inner casing 94 within which or between which an RFID tag 76 is integrated. As previously described in detail above and shown in FIGS. 3-7, the RFID tag 76 includes an RFID chip 78 connected to an RFID antenna 80-with the RFID antenna 80 of RFID tag 76 including two dipoles D1, D2. The RFID tag 76 is positioned and oriented within cover 90 such that a dipole axis, A_{D}, of the RFID tag 76 is approximately aligned with a longitudinal axis, Ac, of the cover 90 (and an axis, As, of the syringe 84). According to embodiments, the dipole axis A_{D} is aligned within +/- 45 degrees of the longitudinal axis Ac of the cover 90 and, more preferably, within +/- 20 degrees of the longitudinal axis Ac of the cover 90 and, still more preferably, is aligned at 0 degrees (i.e., in parallel) to the longitudinal axis Ac of the cover 90. With the dipole axis A_{D} of the RFID tag 76 aligned with the longitudinal axis Ac of the cover 90 (and syringe axis As) in this manner, performance of the RFID tag 76 may be optimized - with reading efficiency of the RFID tag 76 maximized such that the read range of the RFID tag 76 is extended and reading is enabled over a full RFID UHF bandwidth (800 to 1000MHz), and with tag orientation sensitivity being minimized such that the RFID tag 76 provides for omni-directional tag readability all around the syringe axis As (for both ETSI and FCC frequencies). Additionally, with the dipole axis A_{D} of the RFID tag 76 aligned with the longitudinal axis Ac of the cover 60 (and syringe axis As), more strict quality controls may be implemented during manufacturing/assembly of the syringe 84 and the cap 90 thereof, as explained in further detail below.

According to aspects of the disclosure, a method of manufacturing and reading a medical injection device is provided. In some embodiments, the device is in the form of a syringe 10 as illustrated in FIGS. 1 and 2 (or a syringe 84, as illustrated in FIG. 8), as an example. In performing the method, a syringe 10 is provided that includes a hub portion 30 at a distal end 24 thereof. A cover 60 is then coupled to the to the hub portion 30. As previously described, the cover 60 may include an outer casing 62 with an inner wall 72 having a circumference, and an inner casing 64 at least partly inserted within the outer casing 62 and at least partially in contact with the inner wall 72 of the outer casing 62, with the inner casing 64 including an outer wall 74 having a circumference and the inner casing 64 defining a cavity 75 configured to receive at least part of the hub portion 30 and form a seal therewith. An RFID tag 76 is also integrated within the cover 60 during manufacture of the cover 60, with the RFID tag 76 including an RFID chip 78 and an RFID antenna 80 operatively connected to the RFID chip 78. The RFID antenna 80 includes a first tag dipole D1 provided on a first side of the RFID chip 78 and a second tag dipole D2 provided on a second side of the RFID chip 78, with the RFID tag 76 oriented such that the a dipole axis A_{D} of the RFID tag 76 is approximately aligned with a longitudinal axis Ac of the cover 60.

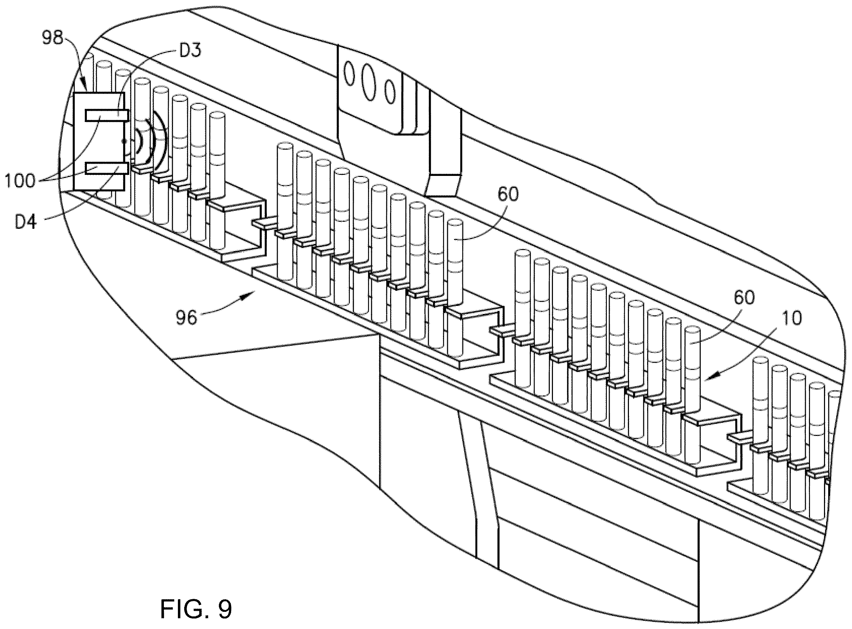

Upon providing/assembly of the syringe 10 (including cover 60), the method may further include steps for testing or interrogation of the RFID tag 76 in cover 60. Referring now to FIG. 9, a manufacturing and reading environment within which the syringe 10 and cover 60 may be tested is illustrated in accordance with an aspect of the disclosure. As shown therein, a conveyer line 96 is provided on and along which a plurality of syringes 10 may be dynamically conveyed/translated as part of an assembly and quality control process. The syringes 10 may be conveyed by conveyer line 96 along a path that is linear or circular. The conveyor line 96 may be operated/constructed as a high throughput conveyer line where the syringes 10 are spaced apart with a pitch of 30mm or less.

As the syringes 10 progress along the conveyor line 96, they pass by an RFID reader 98 configured to read or interrogate the RFID tag 76, so as to determine proper operability of the tag. The RFID reader 98 may include a reader antenna 100 therein that includes a pair of dipoles D3, D4, with the antenna 100 of RFID reader 98 configured such that the dipoles D3, D4 thereof are aligned with the dipoles D1, D2 of the RFID tag 76. Alignment of the RFID reader dipoles D3, D4 with the dipoles D1, D2 of the RFID tag 76 enables reading efficiency of the RFID tag 76 of each syringe 10 to be maximized. That is, with the dipoles D3, D4 of RFID reader 98 aligned with the dipoles D1, D2 of the RFID tag 76, the RFID reader 98 may be structured as an electrical coupling reader antenna for near field application (i.e., near-field electrical antenna) that is able to focus an electrical field toward the syringes 10 that pass thereby. With the RFID reader 98 implementing a near-field electrical antenna 100 that is able to better focus an electrical field on an individual syringe 10 (and RFID tag 76 therein), each individually tagged syringe 10 can be dynamically read without a cross-reading defect potentially occurring - with a cross-reading defect referring to when the tagged syringes 10 are not read in the correct conveying run order (i.e., a 2^{nd} position syringe is read before a 1^{st} position syringe).

Beneficially, embodiments of the invention thus are directed to a cover with an integrated RFID tag that provides an effective and dependable means for traceability of a syringe or other medical injection device, without limitations on visual inspection or data transmission level, and that is not susceptible to damage or that causes challenges in the manufacturing process. The RFID tag is positioned and oriented within the cover such that a dipole axis of the RFID tag is approximately aligned with a longitudinal axis of the cover (and syringe). With the dipole axis aligned with the longitudinal axis of the cover/ syringe in this manner, performance of the RFID tag may be optimized - with reading efficiency of the RFID tag maximized such that the read range of the RFID tag is extended and reading is enabled over a full RFID UHF bandwidth (800 to 1000MHz), and with tag orientation sensitivity being minimized such that the RFID tag provides for omni-directional tag readability all around the syringe axis. Additionally, aligning of the RFID tag dipole axis with the dipole axis of an RFID reader antenna enables reading efficiency of the RFID tag to be maximized, such as by enabling use of an RFID reader near-field electrical antenna that is able to read RFID tags via a focused electrical field directed theretoward. Accordingly, more strict quality controls may be implemented during high throughput manufacturing/assembly of syringes, such as by enabling each individually tagged syringe to be read without a cross-reading defect potentially occurring.

Although the present disclosure has been described in detail for the purpose of illustration based on what is currently considered to be the most practical and preferred embodiments or aspects, it is to be understood that such detail is solely for that purpose and that the present disclosure is not limited to the disclosed embodiments or aspects, but, on the contrary, is intended to cover modifications and equivalent arrangements that are within the spirit and scope of the appended claims. For example, it is to be understood that the present disclosure contemplates that, to the extent possible, one or more features of any embodiment may be combined with one or more features of any other embodiment.

## Claims

1. A cover for use with a medical injection device having a hub portion at a distal end thereof, the cover comprising:
an outer casing including an inner wall having a circumference;
an inner casing at least partly inserted within the outer casing and at least partially in contact with the inner wall of the outer casing, with the inner casing including an outer wall having a circumference and the inner casing defining a cavity configured to receive at least part of the hub portion and form a seal therewith; and
a radio frequency identification (RFID) tag integrated within the cover, the RFID tag comprising:
an RFID chip; and
an RFID antenna operatively connected to the RFID chip, the RFID antenna including a first tag dipole provided on a first side of the RFID chip and a second tag dipole provided on a second side of the RFID chip;
wherein the RFID tag is oriented such that a dipole axis of the RFID tag is approximately aligned with a longitudinal axis of the cover.

2. The cover of claim 1, wherein the dipole axis of the RFID tag comprises an axis extending between a midpoint of the first tag dipole and a midpoint of the second tag dipole.

3. The cover of claim 1, wherein the dipole axis is aligned within +/- 20 degrees with the longitudinal axis of the cover.

4. The cover of claim 1, wherein the dipole axis is aligned at 0 degrees with the longitudinal axis of the cover.

5. The cover of any of claims 1-4, wherein the RFID tag comprises a flat RFID inlay integrated within the cover to match a curvature thereof, with the RFID inlay comprising a first edge and an opposing second edge, with the RFID inlay inlaid within the cover such that a gap is present between the first and second edges.

6. The cover of any of claims 1-5, wherein the RFID tag is located within the inner wall of the outer casing, within the outer wall of the inner casing, or between the outer casing and the inner casing.

7. The cover of any of claims 1-6, wherein the RFID tag is an Ultra High Frequency (UHF) RFID tag.

8. The cover of claim 7, wherein the outer cover is formed of a rigid material and wherein the inner cover is formed of a deformable material.

9. A medical injection device, comprising:
a hub portion; and
a cover according to any of claims 1-12.

10. The medical injection device of claim 9, wherein the medical injection device is a syringe, the syringe including:
a syringe barrel comprising a proximal end and a distal end and defining a chamber, the syringe barrel having an opening at the proximal end and the hub portion at the distal end;
a plunger assembly inserted through the opening and axially movable within the chamber of the syringe barrel, the plunger assembly including:
a plunger rod comprising an elongated body and extending between a proximal end and a distal end; and
a stopper attached to the distal end of plunger rod and positioned within the barrel chamber.

11. A method of manufacturing and reading a medical injection device, the method comprising:
providing a medical injection device including a hub at a distal end thereof;
coupling a cover to the hub, the cover comprising:
an outer casing including an inner wall having a circumference;
an inner casing at least partly inserted within the outer casing and at least partially in contact with the inner wall of the outer casing, with the inner casing including an outer wall having a circumference and the inner casing defining a cavity configured to receive at least part of the hub portion and form a seal therewith; and
a radio frequency identification (RFID) tag integrated within the cover, the RFID tag comprising:
an RFID chip; and
an RFID antenna operatively connected to the RFID chip, the RFID antenna including a first tag dipole provided on a first side of the RFID chip and a second tag dipole provided on a second side of the RFID chip;
wherein the RFID tag is oriented such that the a dipole axis of the RFID tag is approximately aligned with a longitudinal axis of the cover; and
reading the RFID tag via an RFID reader, as the medical injection device and the cover coupled thereto are conveyed along a conveyor line, so as perform a quality control check of the RFID tag.

12. The method of claim 11, further comprising positioning the RFID reader relative to the RFID tag such that a first reader dipole and a second reader dipole of a near-field electrical antenna in the RFID reader are aligned with the first tag dipole and the second tag dipole.

13. The method of claim 12, wherein reading the RFID tag comprises emitting a near-field electrical field from the RFID reader to read the RFID tag.

14. The method of claim 11, wherein in conveying the medical injection device and the cover coupled thereto, a spacing of the medical injection device with other adjacent medical injection devices being conveyed is 30 mm or less, and wherein reading of the RFID tag via the near-field electrical field emitted from the RFID reader prevents cross-reading with one of the other adjacent medical injection devices.

15. The method of claim 14, wherein the dipole axis is aligned within +/- 20 degrees with the longitudinal axis of the cover, and is preferably aligned at 0 degrees with the longitudinal axis of the cover.
